# EUROPEAN PATENT SPECIFICATION

(11) **EP 2 895 451 B1**
(45) Date of publication and mention of the grant of the patent: **14.09.2016**
(21) Application number: 13750417.1
(22) Date of filing: 25.07.2013
(51) Int. Cl.: C07C 29/88, C07C 29/10

(54) **RECOVERY OF TRIMETHYLOLPROPANE FROM PURIFICATION RESIDUE**
RÜCKGEWINNUNG VON TRIMETHYLOLPROPAN AUS REINIGUNGSMITTELRÜCKSTÄNDEN
RÉCUPÉRATION DE TRIMÉTHYLOLPROPANE À PARTIR D'UN RÉSIDU DE PURIFICATION

(30) Priority: 17.09.2012 US 201213621345; 17.09.2012 US 201213621494
(43) Date of publication of application: 22.07.2015
(73) Proprietor: Oxea Bishop LLC, Dallas, TX 75234 (US)
(72) Inventor: WINDHORST, Kenneth, A., Portland, TX 78374 (US); CORTEZ, Oakley, T., Corpus Christi, TX 78414 (US); RAFF, Donald, K., Waynesville, NC 28786 (US)
(74) Representative: Michalski Hüttermann & Partner Patentanwälte mbB
(86) International application number: PCT/US2013/052071
(87) International publication number: WO 2014/042768

(56) References cited:
- WO-A1-01/47848
- DD-A5- 287 251
- DD-A5- 287 252
- US-A- 6 096 905

## Description

### Claim for Priority and Cross Reference to Related Application

This application is based upon United States Patent Application Serial No. 13/621,345, filed September 17, 2012, of the same title, the priority of which is hereby claimed. This application also relates to the subject matter of copending United States Patent Application Serial No. 13/621,494, entitled RECOVERY OF ALCOHOLS FROM PURIFICATION RESIDUE, filed September 17, 2012, the priority of which is also claimed.

### Technical Field

The present invention relates to improved recovery of trimethylolpropane (TMP) from purification residue including TMP-formals by way of acidic hydrolysis in the presence of an oxidizing agent.

### Background

TMP is prepared from n-butyraldehyde and formaldehyde. In one preferred process, base-catalyzed aldol reaction initially generates 2,2-dimethylolbutyraldehyde in a first reaction step which is then converted to a TMP-formate mixture by way of a Cannizzaro reaction. The TMP-containing mixture is typically extracted with an organic solvent, such as ethyl acetate providing an aqueous phase containing the formate and the organic phase comprising TMP. The solvent is separated and the crude TMP is purified by distillation. Typical processing is seen in United States Patent No. 5,603,835 to Cheung et al.*,* Comparative Example 1, Col. 7. *See, also,* United States Patent No. 5,948,943 to Supplee et al.

Purification residue of the TMP production process includes various formals in which TMP equivalents are chemically bound. Such formals may include all or some of the following:
monocyclic TMP-formal (MCF); Formula I: 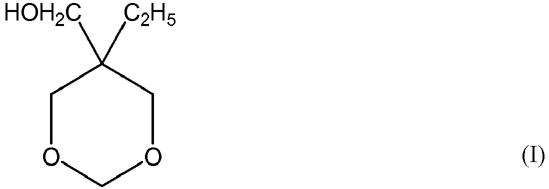
monolinear bis-TMP-formal (MBLF or TMP-BMLF); Formula II:

   [C₂H₅C(CH₂OH)₂CH₂O]₂CH₂ (II)
Methyl-(monolinear)TMP-formal; Formula III

   C₂H₅C(CH₂OH)₂CH₂OCH₂OCH₃ (III)
Methyl-(bislinear)TMP-formal; Formula IV

   C₂H₅C(CH₂OH)₂CH₂OCH₂OCH₂OCH₃ (IV)

Also, typically present in the purification residue are substantial amounts of di-TMP and the cyclic formal of di-TMP; Formula V: 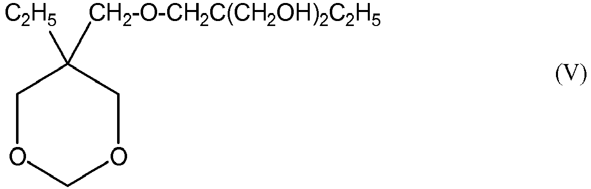 as well as relatively large amounts of TMP. TMP has a structural formula: 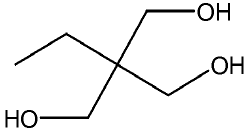 2-(hydroxymethyl)-2-ethylpropane-1,3-diol
and di-TMP has a structural formula: 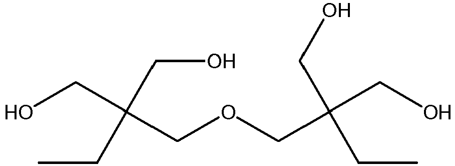 2-[2,2-bis(hydroxymethyl)butoxymethyl]-2-ethylpropane-1,3-diol

In many cases, the main components in the purification residue are TMP, di-TMP and linear bis-TMP-formal (MBLF) (Formula II). Cyclic formals are usually present in smaller amounts.

Because the residues contain substantial amounts of TMP, either physically mixed or bound in formal equivalents, and also di-TMP, which is also a valuable product for resins, lubricant oils or the like, various processes to recover TMP and di-TMP from residues have been developed.

United States Patent No. 7,301,058 to Wartini et al. discloses a process for recovering TMP from purification residue using acid treatment in water. Increases in TMP levels of from -6.4% to 36.3% are reported in Table 1, Col. 9. DD 28 72 51 likewise refers to cleavage of TMP formals under acidic conditions followed by thermal treatment.

United States Patent No. 6,265,623 to Moraweitz et al. relates to the reductive cleavage of linear and cyclic acetals, especially formals, in an aqueous medium containing a formate which takes place by hydrogenation with hydrogen in the presence of a heterogeneous hydrogenation catalyst at a pH value of less than 7 at a temperature of over 200°C. *See, also,* WO 97/01523. Under these reaction conditions, formaldehyde liberated from the formals is hydrogenated to methanol. A similar hydrogenation process of the TMP residue is disclosed in DE 10 2010 033 844. The aqueous solution from the hydrogenation step is freed from the catalyst and other solids and then treated with ion exchangers. From the collected solution, light ends are removed and then a TMP-enriched head fraction is recovered.

United States Patent Application Publication No. US 2002/0033325 of Ninomiya et al. teaches to add an acid to TMP purification residue and to add a scavenger for the formaldehyde liberated in the acid treatment. Said scavenger, such as hydroxylamine salts, avoids the formation of cyclic formals, such as the cyclic formal of TMP and the cyclic formal of di-TMP.

In United States Patent No. 6,316,679 to Supplee et al. there is disclosed a process for treating TMP heavy ends residue with a strong acid in the presence of an alcohol formaldehyde scavenger. Increases in TMP content from 10% to 30% are reported. *See* Table III, Col. 6. Similar features appear in United States Patent No. 6,096,905, also to Supplee et al.

### Summary of Invention

We have found that unexpectedly high yield of TMP from conversion of TMP-formals is achieved by treatment of TMP purification residue with an acid in the presence of an oxidizing agent. Without intending to be bound by any particular theory, it is believed the oxidizing agent inhibits or prevents cyclization of formals. The formaldehyde liberated in the cleavage of the TMP-formals is oxidized to formic acid and the concurrent formation of cyclic formals of TMP is substantially reduced or eliminated. In consequence, the efficiency of the TMP production process can be substantially improved. Typically, a two-step process is employed which converts TMP bis linear formal (MBLF) to TMP and potassium formate:

(TMP bis linear formal) + H₂O₂ + H₂O ---- H⁺ ---> 2 - x TMP + x TMP formate + formic acid (+ traces of MCF)

and :

TMP formate + formic acid + KOH ----> TMP + KFo (potassium formate)

KFo is extracted away from the TMP using an ethyl acetate multistage extraction process in a preferred embodiment.

Preferred acids include strong mineral acids such as sulfuric acid, phosphoric acid, hydrochloric acid, sulfonic acid and the like or organic acids, such as formic acid, acetic acid, or oxalic acid. Preferably formic acid or an acid-functional ion exchange resin is selected. Particularly preferred are sulfonic acid functional ion exchange resins. The acidic compound is added to the aqueous medium in an amount such that the pH value is usually less than 4, typically in the range of from 1 to 4 and in particular in the range of from 1.5 to 3. As oxidation reagent oxygen gas, ozone, oxygen containing gas, ozone containing gas, peracids such as peracetic acid or aqueous hydrogen peroxide and the like can be used. Preference is given to an aqueous hydrogen peroxide solution. Hydrogen peroxide will be reduced to water and no additional component will be formed during this oxidizing treatment.

The inventive method provides a simple and cost-saving process to improve the overall TMP efficiency. The prior art teaches in some aspects hydrogenation in the presence of a hydrogenation catalyst which makes the known process more complicated and requires additional catalytic material including its handling. Further, the use of various scavengers as known in the art adds an additional auxiliary component, which reaction product from the formaldehyde scavenging process has to be separated.

Using an oxidizing reagent has the advantage that formaldehyde liberated will be oxidized to formic acid which can be converted into alkali or alkaline earth metal formate after neutralization of the treated purification residue with an aqueous solution of said metal compounds. The recovered aqueous formate solution can be combined with process streams if the reaction of n-butyraldehyde with formaldehyde is carried out as an inorganic Cannizzaro process which produces formates. In any event, the inventive method can optionally be applied for heavies from any TMP production process, such as a process for making TMP by way of reacting butyraldehdye with formaldehyde followed by hydrogenation as described in United States Patent No. 7,301,058 to Wartini et al. noted above. Likewise, other formals and acetals can be recovered by way of the inventive process as described hereinafter.

Further details and advantages will become apparent from the discussion which follows.

### Description of Drawings

The invention is described in detail below in connection with numerous examples and in connection with the attached **Figures.** In the **Figures:**
**Figure 1** is a schematic diagram illustrating continuous operation of a reactor recovering polyols from purification residue in accordance with the invention;
**Figure 2** is a simplified schematic diagram of a polyol reclamation system of the invention integrated with a conventional product recovery system; and
**Figure 3** is a simplified schematic diagram of another alcohol reclamation system of the invention integrated with a conventional product recovery system.

### Detailed Description

The invention is described in detail below in connection with the Figures for purposes of illustration, only. The invention is defined in the appended claims. Terminology used throughout the specification and claims herein is given its ordinary meaning as supplemented by the discussion immediately below, for example, "conversion", " selectivity" and yield are related by the mathematical definition X(conversion) * S(selectivity) = Y(yield), all calculated on a weight or molar basis; e.g. in a certain reaction, 90% of substance A is converted (consumed), but only 80% of it is converted to the desired substance B and 20% to undesired by-products, so conversion of A is 90%, selectivity for B 80% and yield of substance B is 72% (= 90% * 80%).

For purposes of calculating weight ratios or percentages, "Organic", "organic mixture" and like terminology refers to the subject composition or component on a dry basis unless the context indicates otherwise.

Unless otherwise indicated, "percent", "%" or like terminology refers to weight percent of a component. Likewise, "parts" refers to parts by weight unless otherwise indicated.

Residence time in a continuous reactor is calculated as the steady state or average volume of the reaction medium in the reactor divided by the volumetric flow rate through the reactor.

Reaction rates and yields are especially favorable with respect to linear formal residues of TMP as described below.

### Examples 1-5

TMP was prepared from n-butyraldehyde and formaldehyde by way of a base-catalyzed aldol reaction initially generating 2,2-dimethylolbutyraldehyde in a first reaction step which was then converted to a TMP and TMP-formate mixture by way of a Cannizzaro reaction. The TMP-containing mixture was extracted with ethyl acetate providing an aqueous phase containing the formate and the organic phase comprising TMP. The solvent was separated and the crude TMP was purified by distillation as described generally in United States Patent No. 5,603,835 to Cheung et al.*,* Comparative Example 1, Col. 7. The distillation (purification) residue or "heavies" was used as feed to the inventive process. In Tables 1 and 2, the heavies are referred to as starting materials and analyses are provided on a dry basis for the purification residues and the products, excluding water, oxidizing agent, and acid which concentrations are reported on a wet basis as present in the aqueous reaction mixture in Tables 1 and 2.

The procedure followed for Examples 1-5 was to mix about 65 grams of TMP heavies (analyses as per Tables 1, 2) with from about 130 to 260 grams of water (2/1 up to 4/1 water to organics ratio), from about 8 to about 20 grams of formic acid (sometimes referred to herein as Hfo or HFO) or a corresponding amount of sulfonic acid resin, from about 7 to about 20 grams of hydrogen peroxide (100% basis, reported wet in Tables 1 and 2). Anywhere from 3% H₂O₂ up to 30% based on the dry weight of starting material has little effect on the process. The water in the H₂O₂ solution is included in the water added and included in the percentage of acid and H₂O₂ in Tables 1 and 2. The reaction mixture was shaken to make it homogenous and then heated to 70°C to 90°C for various lengths of time (2-4 hours) to achieve conversion of MBLF. The aqueous medium is optionally neutralized with potassium hydroxide to provide potassium formate which may be combined with formate generated in the Cannizzaro synthesis, as noted above, after further work-up. Details and results appear in Tables 1, 2 below.

Further work-up includes extracting formates from the reclaimed TMP and distillation of the TMP to purified form as is known in the art.

**Table 1**

| | | | **Example 1** | **Example 2** | | **Example 3** |
|---|---|---|---|---|---|---|
| **Component** | **Starting Material** | | **Treated** | **Treated and Neutralized** | | **Treated** |
| | | | **1% H₂O₂ + 4% Hfo** | **1%H₂O₂ + 4% Hfo Neutralized, pH=9+** | | **%H₂O₂ +6%A16 resin**** |
| % MCF | 0 | | 3.5 | 3.51 | | 11.51 |
| % TMP | 15.66 | | 46.88 | 55.84 | | 54.13 |
| % TMP formates | 0 | | 14.1 | 3.77 | | 5.7 |
| % DMB* | 5.56 | | 7.44 | 8.87 | | 8.79 |
| % Di TMP | 15.26 | | 9.39 | 12.65 | | 11.49 |
| % MBLF | 54.15 | | 4.19 | 5.39 | | 0.08 |
| Unknowns | 9.37 | | 14.5 | 9.97 | | 8.3 |
| | | | | | | **pH** = **2.6** |
| MBLF Conversion | | | 92.26 | 90.05 | | 99.85 |
| TMP yield (weight) | #TMP/#MBLF consumed | | 0.62 | 0.82 | | 0.71 |
| MCF yield (weight) | #MCF/#MBLF consumed | | 0.07 | 0.07 | | 0.21 |
| % molar TMP selectivity from MBLF | | | 65.29 | 86.09 | | 74.33 |

| | | | | | | |
|---|---|---|---|---|---|---|
| *dimethylolbutyraldehyde **sulfonic acid functional macroreticular ion exchange resin | | | | | | |

**Table 2**

| | | | **Example 4** | **Example 5** |
|---|---|---|---|---|
| **Component** | **Starting Material** | | **Treated** | **Treated and Neutralized** |
| | | | **2% H₂O₂ + 4% Hfo** | **2%H₂O₂ + 4% Hfo** |
| | | | | **Neutralized (longer)** |
| % MCF | 0 | | 3.97 | 3.81 |
| % TMP | 11.07 | | 56.91 | 63.22 |
| % TMP formates | 0 | | 12.7 | 2.87 |
| % DMB | 5.9 | | 5.98 | 7.27 |
| % Di TMP | 15.05 | | 10.49 | 13.33 |
| % MBLF | 58.97 | | 0.37 | 0.49 |
| Unknowns | 9.01 | | 9.58 | 9.01 |
| | | | | |
| MBLF Conversion | | | 99.37 | 99.17 |
| TMP yield (Weight) | #TMP/#MBLF consumed | | 0.78 | 0.89 |
| MCF yield (weight) | #MCF/#MBLF consumed | | 0.07 | 0.07 |
| | | | | |
| % molar TMP yield from MBLF | | | 81.73 | 93.17 |

### Examples 6-13

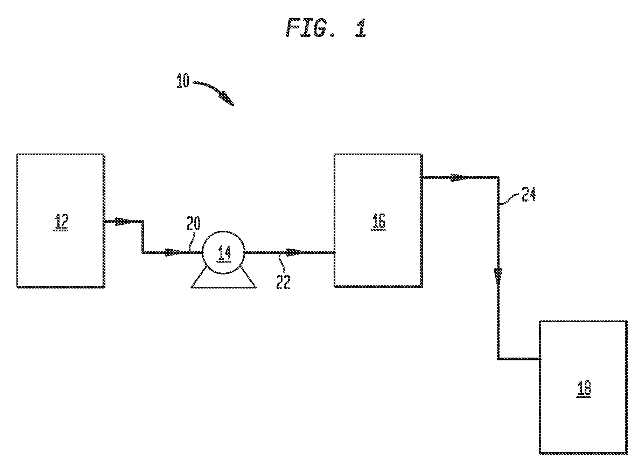

Additional trials were carried out in an apparatus **10** shown schematically in **Figure 1****.** The reaction system included a stirred vessel **12,** a pump **14,** a heated overflow, continuous (plug flow type) reactor **16** and a collection vessel **18.** In operation, starting material having the composition generally as in Examples 2, 4 and 5 was charged to stirred vessel **12,** along with water, formic acid and hydrogen peroxide, in amounts as in Examples 1-5. The reactants were homogenized in stirred vessel **12** and continuously fed to reactor **16** via lines **20, 22** and pump **14** wherein the reactants were maintained at reaction temperature for a residence time duration of 2-6 hours. Overflow from reactor **16** after the aforementioned residence time in the reactor was continuously collected in vessel **18** by way of line **24.** The material was neutralized as noted in Examples 2, 4 and 5 and analyzed for conversion of MBLF to TMP. The effects of temperature and reaction time on MBLF conversion to TMP appear in Table 3.

**Table 3: Reaction Time, Temperature Results**

| Reactor Temperature °C | Reactor Residence Time, Hours | MBLF Conversion (wt. %) |
|---|---|---|
| 60 | 2 | 7% |
| | 4 | 15% |
| | 6 | 21% |
| 75 | 2 | 39% |
| | 4 | 61% |
| | 6 | 74% |
| 90 | 2 | 92% |
| | 4 | 97% |

Suitable and preferred process features are listed in Table 4 below.

**Table 4: Process Features**

| **Process Feature** | **Typical Range(s)** | **Preferred Range(s)** |
|---|---|---|
| **Hydrolysis Temperature °C** | 50°C - 99°C | 70°C - 95°C |
| **Reaction Time; Residence Time in Reactor under Reaction Conditions, hours** | 0.5 - 10 | 1.5 - 4.5 |
| | 1 - 7.5 | |
| **Oxidizing Agent; % by Weight Based on Dry Starting Material** | 2% - 40% | 5% - 10% |
| | 4% - 20% | |
| **Acid Concentration; % by Weight of Hydrolysis Medium Wet Basis** | 0.5% - 20% | 2% - 10% |
| | 1% - 15% | |
| **pH of Reaction Medium Prior to Neutralization** | less than 4 | 1.5 to 3 |
| | 1 to 4 | |
| **Water/Organic Ratio W/W** | 0.5 to 4 | 1.5 to 2.5 |
| | 1-3 | |
| **Yield, Weight Basis; Weight of polyhydric alcohol reclaimed per weight of polyhydric alcohol derivate in feed, %** | 50% - 99% | 75% - 95% |
| | 60% - 98% | >80% |

### Example 14 - Integration with Conventional TMP Recovery

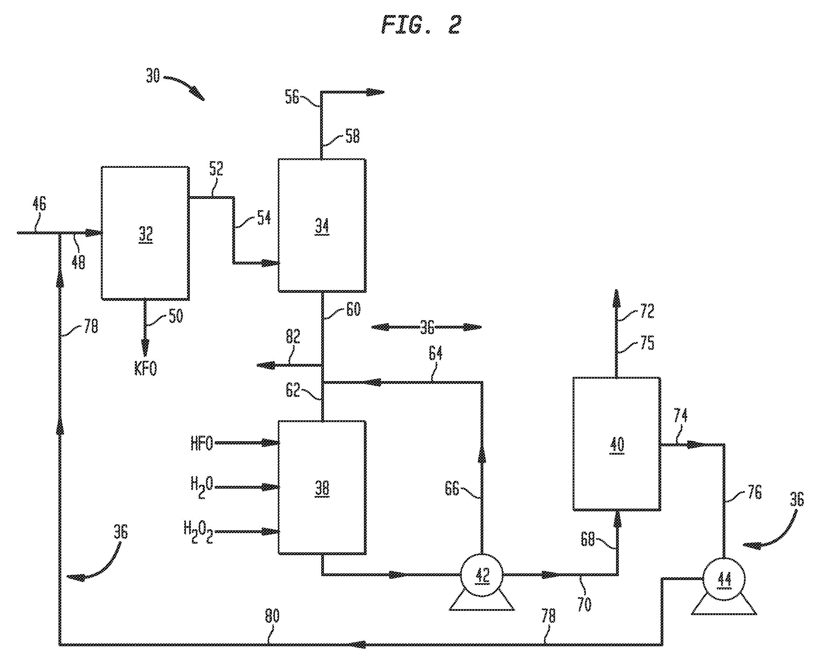

The process of the present invention may be integrated into an existing TMP recovery process as shown schematically in the apparatus of **Figure 2****.**

**Figure 2** shows an apparatus **30** including a multistage water/ethyl acetate extraction system **32,** a distillation tower **34** and a residue recovery recycle loop **36.** Loop **36** includes a mixing system **38,** a heated hydrolysis reactor **40,** as well as pumps **42, 44** and a plurality of connecting conduits therebetween.

Extraction system **32** and purification tower **34** may be of conventional design wherein a TMP/formate crude product stream **46** is fed to system **32** via line **48** where potassium formate is extracted in an aqueous phase and recovered as stream **50.** TMP is recovered from system **32** in an organic phase **52** and fed to tower **34** via line **54** wherein TMP is taken together with light ends as an overhead stream **56** via line **58** and directed to further purification (not shown).

Distillation residue exits tower **34** as stream **60** and is fed to mixing system **38** via line **62.** In mixing system **38** hydrogen peroxide, water and formic acid are added to the purification residue and the resulting mixture is partially recycled to system **38** as stream **64** via line **66** by way of pump **42.** Any equivalent means of providing hydrogen peroxide, water and acid to the reaction mixture are also contemplated, for example, these components may be admixed before or provided directly to reactor **40.**

Pump **42** also provides a reactant stream **68** including purification residue, formic acid and hydrogen peroxide to hydrolysis and oxidation reactor **40** via line **70.** In reactor **40,** TMP is recovered from stream **68** as described above in connection with Examples 1-13 and any excess H₂O₂ is vented to a scrubber as stream **72** via line **75.**

Reactor **40** is a plug flow type overflow reactor. A reacted stream 74 is enriched in TMP by the alcohol recovery process of the invention and the liquor exits via line **76** to pump **44** which pumps the material as stream **78** via line **80** to feed line **48** where it is recycled to extraction system **32.**

The alcohol reclaimed from the purification residue is thus returned to an existing purification system where it is recovered along with product from a fresh raw product stream. If so desired, one could treat and recycle recovered material directly to purification tower **34.**

As system **30** operates, levels of heavy by-products in stream **60** increase because of the operation of residue cycle loop **36.** A purge stream **82** is withdrawn from stream 60 and may be further processed or incinerated for steam generation.

### Example 15 - Integration with Existing TMP Distillation

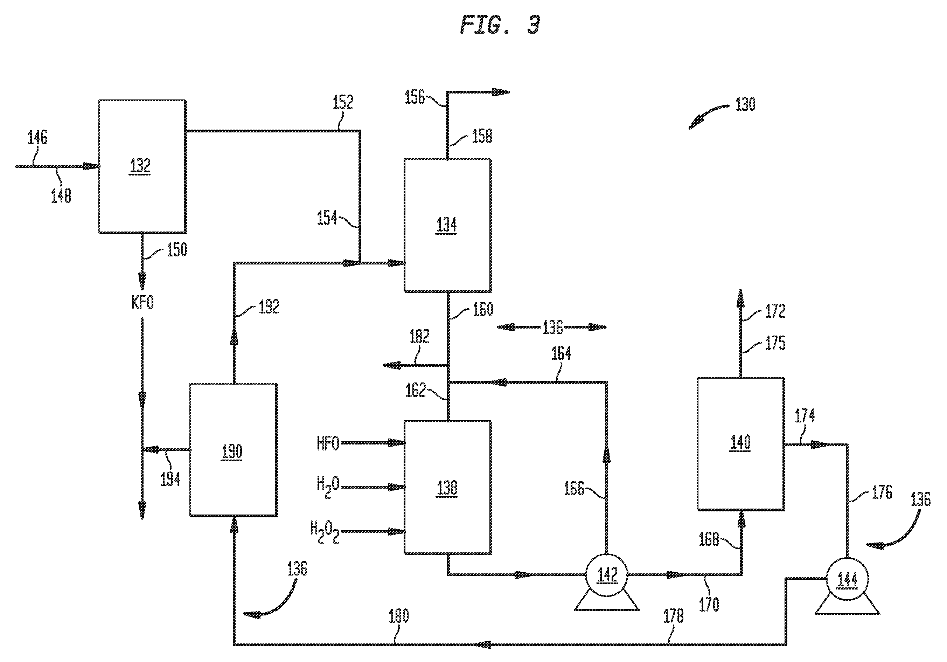

Another embodiment showing integration of the inventive recovery system is shown in **Figure 3** which provides a system **130** including a multistage water/ethyl acetate extraction system **132,** a distillation tower **134** and a residue recovery recycle loop **136.** Loop **136** includes a mixing system **138,** a heated hydrolysis reactor **140,** an extraction/ neutralization unit **190** as well as pumps **142, 144** and a plurality of connecting conduits therebetween.

Extraction system **132** and purification tower **134** may be of conventional design as noted above in connection with **Figure 2** wherein a TMP/formate crude product stream **146** is fed to system **132** via line **148** where potassium formate is extracted in an aqueous phase and recovered as stream **150.** TMP is recovered from system **132** in an organic phase **152** and fed to tower **134** via line **154** wherein TMP is taken together with light ends as an overhead stream **156** via line **158** and directed to further purification.

Distillation residue exits tower **134** as stream **160** and is fed to mixing system **138** via line **162.** In mixing system **138** hydrogen peroxide, water and formic acid are added to the purification residue and the resulting mixture is partially recycled to system **138** as stream **164** via line **166** by way of pump **142.** Any equivalent means of providing hydrogen peroxide, water and acid to the reaction mixture are also contemplated, for example, these components may be admixed before or provided directly to reactor **140.**

Pump **142** also provides a reactant stream **168** including purification residue, formic acid and hydrogen peroxide to hydrolysis and oxidation reactor **140** via line **170.** In reactor **140,** TMP is recovered from stream **168** as described above in connection with Examples 1-13 and any excess H₂O₂ is vented to a scrubber as stream **172** via line **175.**

Reactor **140** is a plug flow type overflow reactor. A reacted stream **174** is enriched in TMP by the alcohol recovery process of the invention and the liquor exits via line **176** to pump **144** which pumps the material as stream **178** via line **180** to extraction/ neutralization unit **190** where formic acid in the effluent from reactor **140** is neutralized to potassium formate and exits the unit as stream **194** which is combined with stream **150.** Reclaimed TMP exits unit **190** as an organic stream **192** which is combined with organic phase **152** and fed to tower **134.**

The alcohol reclaimed from the purification residue is thus returned to an existing purification tower where it is recovered along with product from a fresh raw product stream.

As system **130** operates, levels of heavy by-products in stream **160** increase because of the operation of residue cycle loop **136** and are further processed or incinerated for steam generation as noted above in connection with **Figure 2****.** To this end, a purge stream **182** is drawn from stream **160** as shown.

There is thus provided in a first Embodiment, a method of reclaiming trimethylolpropane from purification residue comprising hydrolyzing a purification residue with an aqueous medium in the presence of (i) an acid and (ii) an oxidizing agent to reclaim trimethylolpropane from one or more TMP-formals selected from:
monocyclic TMP-formal (MCF); Formula I: 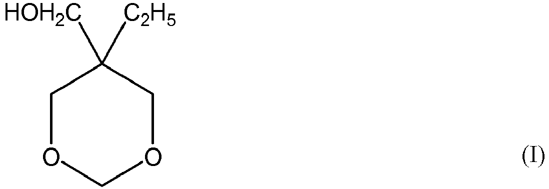
monolinear bis-TMP-formal (MBLF); Formula II:

   [C₂H₅C(CH₂OH)₂CH₂O]₂CH₂ (II)
Methyl-(monolinear)TMP-formal; Formula III

   C₂H₅C(CH₂OH)₂CH₂OCH₂OCH₃ (III)
Methyl-(bislinear)TMP-formal; Formula IV

   C₂H₅C(CH₂OH)₂CH₂OCH₂OCH₂OCH₃ (IV)
and the cyclic formal of di-TMP; Formula V:

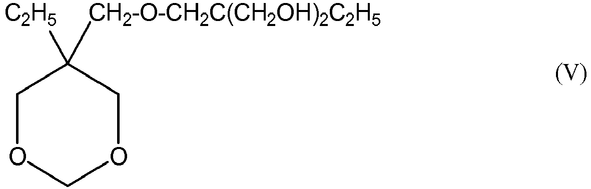

The following are additional embodiments:
Embodiment No. 2 is the method according to Embodiment No. 1, comprising hydrolyzing
   monolinear bis-TMP-formal (MBLF); Formula II:

   [C₂H₅C(CH₂OH)₂CH₂O]₂CH₂ (II)

   in the presence of (i) an acid and (ii) an oxidizing agent.
Embodiment No. 3 is the method according to Embodiment No. 1 or Embodiment No. 2, wherein said acid is selected from sulfuric acid, phosphoric acid, hydrochloric acid, sulfonic acid, organic acids or acid-functional ion exchange resin.
Embodiment No. 4 is the method according to Embodiment No. 1 and any or all of the other foregoing Embodiments, wherein said acid is formic acid.
Embodiment No. 5 is the method according to Embodiment No. 3, wherein said acid is an acid functional ion exchange resin.
Embodiment No. 6 is the method according to Embodiment No. 5, wherein said ion exchange resin is a sulfonic acid functional ion exchange resin.
Embodiment No. 7 is the method according to Embodiment No. 1 and any or all of the other foregoing Embodiments, wherein the hydrolysis reaction is carried out at a pH of less than 4.
Embodiment No. 8 is the method according to Embodiment Nos. 1 through 6, wherein the hydrolysis reaction is carried out at a pH of from 1 to 4.
Embodiment No. 9 is the method according to Embodiment No. 8, wherein the hydrolysis reaction is carried out at a pH of from 1.5 to 3.
Embodiment No. 10 is the method according to Embodiment No. 1 and any or all of the other foregoing Embodiments, further comprising neutralizing said aqueous medium with a neutralizing agent.
Embodiment No. 11 is the method according to Embodiment No. 10 wherein said acid is formic acid and said neutralizing agent is an alkali metal hydroxide.
Embodiment No. 12 is the method according to Embodiment No. 1 and any or all of the other foregoing Embodiments, effective to reclaim at least 75% by weight of the trimethylolpropane present in the trimethylolpropane formals of the purification residue.
Embodiment No. 13 is the method according to Embodiment No. 1 and any or all of the other foregoing Embodiments, effective to reclaim at least 80% by weight of the trimethylolpropane present in the trimethylolpropane formals of the purification residue.
Embodiment No. 14 is the method according to Embodiment No. 1 and any or all of the other foregoing Embodiments, effective to reclaim at least 85% by weight of the trimethylolpropane present in the trimethylolpropane formals of the purification residue.
Embodiment No. 15 is the method according to Embodiment No. 1 and any or all of the other foregoing Embodiments, effective to reclaim at least 90% by weight of the trimethylolpropane present in the trimethylolpropane formals of the purification residue.
Embodiment No. 16 is the method according to Embodiment No. 1 and any or all of the other foregoing Embodiments, wherein the oxidizing agent is supplied to the hydrolysis reaction in an amount of from 2 wt% to 40 wt% based on the dry weight of the purification residue.
Embodiment No. 17 is the method according to Embodiment No. 1 and any or all of the other foregoing Embodiments, wherein the oxidizing agent is supplied to the hydrolysis reaction in an amount of from 4 wt% to 20 wt% based on the dry weight of the purification residue.
Embodiment No. 18 is the method according to Embodiment No. 17, wherein the oxidizing agent is supplied to the hydrolysis reaction in an amount of from 5 wt% to 10 wt% based on the dry weight of the purification residue.
Embodiment No. 19 is the method according to Embodiment No. 1 and any or all of the other foregoing Embodiments, wherein the acid is supplied to the aqueous reaction medium in an amount of from 0.5 wt% to 20 wt% based on the total weight of the aqueous medium.
Embodiment No. 20 is the method according to Embodiment No. 19, wherein the acid is supplied to the aqueous reaction medium in an amount of from 1 wt% to 15 wt% based on the total weight of the aqueous medium.
Embodiment No. 21 is the method according to Embodiment No. 20, wherein the acid is supplied to the aqueous reaction medium in an amount of from 2 wt% to 10 wt% based on the total weight of the aqueous medium.
Embodiment No. 22 is the method according to Embodiment No. 1 and any or all of the other foregoing Embodiments, wherein water is supplied to the reaction medium in a weight ratio of water: organic mixture from 0.5 W/W to 4 W/W.
Embodiment No. 23 is the method according to Embodiment No. 22, wherein water is supplied to the reaction medium in a weight ratio of water:organic mixture from 1 W/W to 3 W/W.
Embodiment No. 24 is the method according to Embodiment No. 23, wherein water is supplied to the reaction medium in a weight ratio of water:organic mixture from 1.5 W/W to 2.5 W/W.
Embodiment No. 25 is the method according to Embodiment No. 1 and any or all of the other foregoing Embodiments, wherein the hydrolysis temperature is maintained within the range of from 50°C to 99°C.
Embodiment No. 26 is the method according to Embodiment No. 25, wherein the hydrolysis temperature is maintained within the range of from 70°C to 95°C.
Embodiment No. 27 is the method according to Embodiment No. 1 and any or all of the other foregoing Embodiments, wherein the hydrolysis reaction is carried out for a reaction period of from about 0.5 hours to about 10 hours.
Embodiment No. 28 is the method according to Embodiment No. 27, wherein the hydrolysis reaction is carried out for a reaction period of from about 1 hour to about 7.5 hours.
Embodiment No. 29 is the method according to Embodiment No. 28, wherein the hydrolysis reaction is carried out for a reaction period of from about 1.5 hours to about 4.5 hours.
Embodiment No. 30 is the method according to Embodiment No. 1 and any or all of the other foregoing Embodiments, wherein reactants, temperature and reaction time are selected such that the yield of trimethylolpropane is from 50% to 99% based on the weight of TMP-formal derivate in the purification residue.
Embodiment No. 31 is the method according to Embodiment No. 30, wherein reactants, temperature and reaction time are selected such that the yield of trimethylolpropane is from 60% to 98% based on the weight of TMP-formal derivate in the purification residue.
Embodiment No. 32 is the method according to Embodiment No. 30, wherein reactants, temperature and reaction time are selected such that the yield of trimethylolpropane is from 75% to 95% based on the weight of TMP-formal derivate in the purification residue.
Embodiment No. 33 is the method according to Embodiment No. 32, wherein reactants, temperature and reaction time are selected such that the yield of trimethylolpropane is greater than 80% based on the weight of TMP formal derivate in the purification residue.
Embodiment No. 34 is a process for reclaiming trimethylolpropane from purification residue comprising: (a) continuously feeding a purification residue stream to a reaction vessel, said purification residue stream containing one or more trimethylolpropane formals selected from:
   monocyclic TMP-formal (MCF); Formula I: 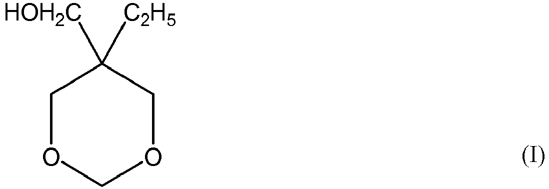
   monolinear bis-TMP-formal (MBLF); Formula II:

      [C₂H₅C(CH₂OH)₂CH₂O]₂CH₂ (II)
   Methyl-(monolinear)TMP-formal; Formula III

      C₂H₅C(CH₂OH)₂CH₂OCH₂OCH₃ (III)
   Methyl-(bislinear)TMP-formal; Formula IV

      C₂H₅C(CH₂OH)₂CH₂OCH₂OCH₂OCH₃ (IV)
   and formal of di-TMP; the cyclic Formula V: 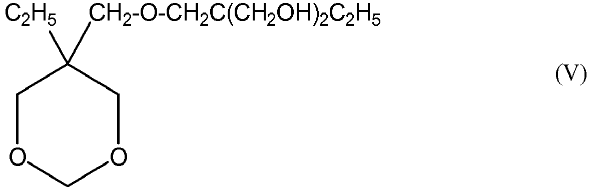
   (b) providing water, an acid and an oxidizing agent to the reaction vessel such that a reaction mixture including the purification residue, water, acid and oxidizing agent is formed; (c) maintaining the reaction mixture in the reaction vessel at a time and temperature such that one or more of said TMP-formals are hydrolyzed to thereby enrich the reaction mixture with reclaimed trimethylolpropane; and (d) continuously withdrawing a reclaimed trimethylolpropane stream from the reaction vessel. This embodiment many utilize any of, or all of, the features of Embodiments 1-33.
Embodiment No. 35 is the process according to Embodiment No. 34, wherein the reclaimed trimethylolpropane stream from step (d) is subjected to a distillation to obtain trimethylolpropane containing light ends and a distillation residue.
Embodiment No. 36 is the method according to Embodiment No. 35, wherein the reclaimed trimethylolpropane stream is combined with another trimethylolpropane stream to provide a combined trimethylolpropane stream prior to distillation.
Embodiment No. 37 is the method according to Embodiment No. 36, wherein formate is extracted from the combined trimethylolpropane stream prior to distillation.
Embodiment No. 38 is a process for purifying a trimethylolpropane containing stream comprising: (a) distilling a trimethylolpropane containing stream to provide an overhead product stream and a purification residue stream; (b) feeding the purification residue stream to a reaction vessel, said purification residue stream containing one or more trimethylolpropane formals selected from:
   monocyclic TMP-formal (MCF); Formula I: 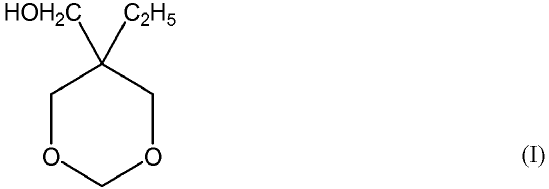
   monolinear bis-TMP-formal (MBLF); Formula II:

      [C₂H₅C(CH₂OH)₂CH₂O]₂CH₂ (II)
   Methyl-(monolinear)TMP-formal; Formula III

      C₂H₅C(CH₂OH)₂CH₂OCH₂OCH₃ (III)
   Methyl-(bislinear)TMP-formal; Formula IV

      C₂H₅C(CH₂OH)₂CH₂OCH₂OCH₂OCH₃ (IV)
   and formal of di-TMP; the cyclic Formula V: 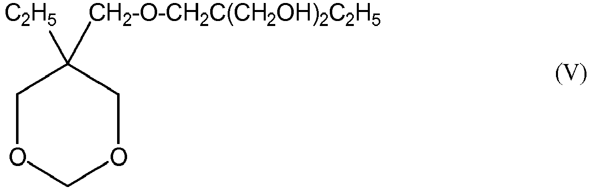
   (c) continuously providing water, an acid and an oxidizing agent to the reaction vessel such that a reaction mixture including the purification residue, water, acid and oxidizing agent is formed; (d) maintaining the reaction mixture in the reaction vessel at a time and temperature such that one or more of said trimethylol formals are hydrolyzed to thereby enrich the reaction mixture with reclaimed trimethylolpropane; (e) withdrawing a reclaimed trimethylolpropane stream from the reaction vessel; and (f) recycling reclaimed trimethylolpropane to step (a). This embodiment many utilize any of, or all of the features of Embodiments 1-37.
Embodiment No. 39 is the method according to Embodiment No. 38, wherein an aqueous phase is extracted from the reclaimed trimethylolpropane stream prior to recycling reclaimed trimethylolpropane to step (a).
Embodiment No. 40 is a purification system for recovering trimethylolpropane from a raw product stream comprising trimethylolpropane and formate comprising: (a) an extraction system adapted to receive the raw product stream and provide aqueous output containing formate and an organic output containing trimethylolpropane; (b) a distillation tower adapted to receive organic output of the extraction system and provide trimethylolpropane and light ends as overhead and purification residue, wherein the purification residue contains one or more TMP-formals selected from:
   monocyclic TMP-formal (MCF); Formula I: 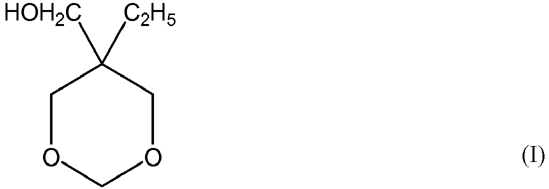
   monolinear bis-TMP-formal (MBLF); Formula II:

      [C₂H₅C(CH₂OH)₂CH₂O]₂CH₂ (II)
   Methyl-(monolinear)TMP-formal; Formula III

      C₂H₅C(CH₂OH)₂CH₂OCH₂OCH₃ (III)
   Methyl-(bislinear)TMP-formal; Formula IV

      C₂H₅C(CH₂OH)₂CH₂OCH₂OCH₂OCH₃ (IV)

      and the cyclic formal of di-TMP; Formula V: 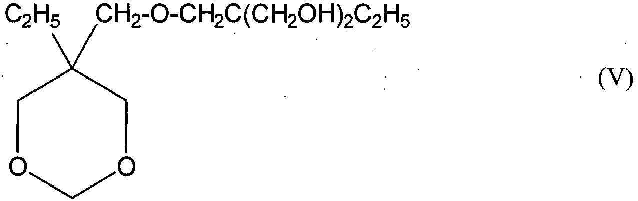
   (c) means for providing water, an oxidizing agent, and acid to the purification residue to provide an aqueous residue mixture; (d) a reactor adapted for receiving the aqueous residue mixture and maintaining the reaction mixture in the reaction vessel at a time and temperature such that one or more of said TMP-formals are hydrolyzed to thereby enrich the reaction mixture with reclaimed trimethylolpropane; and (e) a recycle system for recycling reclaimed trimethylolpropane from the reactor vessel to the extraction system of part (a) or for recycling reclaimed trimethylolpropane from the reactor to the distillation tower of part (b). Any of, or all of the features of Embodiments 1-39 may be employed in connection with the system of this embodiment.

## Claims

1. A method of reclaiming trimethylolpropane from purification residue comprising hydrolyzing a purification residue with an aqueous medium in the presence of (i) an acid and (ii) an oxidizing agent to reclaim trimethylolpropane from one or more TMP-formals selected from:
monocyclic TMP-formal (MCF); Formula I: 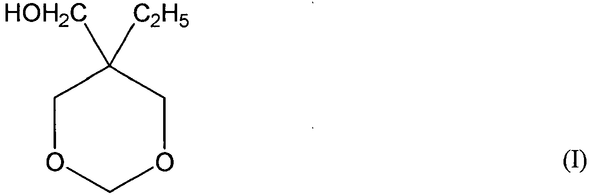
monolinear bis-TMP-formal (MBLF); Formula II:
[C₂H₅C(CH₂OH)₂CH₂O]₂CH₂ (II)
Methyl-(monolinear)TMP-formal; Formula III
C₂H₅C(CH₂OH)₂CH₂OCH₂OCH₃ (III)
Methyl-(bislinear)TMP-formal; Formula IV
C₂H₅C(CH₂OH)₂CH₂OCH₂OCH₂OCH₃ (IV)
and the cyclic formal of di-TMP; Formula V: 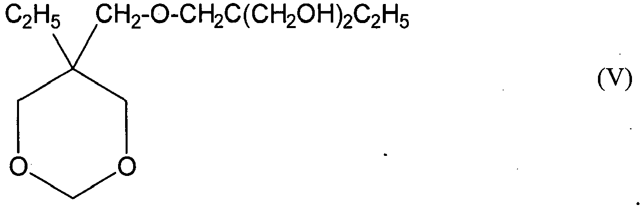 wherein the oxidizing agent is supplied to the hydrolysis reaction in an amount of from 2 wt% to 40 wt% based on the dry weight of the purification residue.

2. The method according to Claim 1, comprising hydrolyzing monolinear bis-TMP-formal (MBLF); Formula II:
[C₂H₅C(CH₂OH)₂CH₂O]₂CH₂ (II)
in the presence of (i) an acid and (ii) an oxidizing agent.

3. The method according to Claim 1, wherein said acid is selected from sulfuric acid, phosphoric acid, hydrochloric acid, sulfonic acid, organic acids or acid-functional ion exchange resin.

4. The method according to Claim 1, wherein said acid is formic acid.

5. The method according to Claim 1, wherein said acid is an acid functional ion exchange resin.

6. The method according to Claim 1, wherein the hydrolysis reaction is carried out at a pH of from 1 to 4.

7. The method according to Claim 1, further comprising neutralizing said aqueous medium with a neutralizing agent.

8. The method according to Claim 1, wherein the hydrolysis reaction is carried out for a reaction period of from 1.5 to 4.5 hours.

9. The method according to Claim 1, wherein the acid is supplied to the aqueous reaction medium in an amount of from 0.5 wt% to 20 wt% based on the total weight of the aqueous medium.

10. The method according to Claim 1, wherein water is supplied to the reaction medium in a weight ratio of water:organic mixture from 1 W/W to 3 W/W.

11. The method according to Claim 1, wherein the hydrolysis temperature is maintained within the range of from 70°C to 95°C.

12. The method according to Claim 1, wherein the hydrolysis reaction is carried out for a reaction period of from 1 hour to 7.5 hours.

13. The method according to Claim 1, wherein the oxidizing agent is supplied to the hydrolysis reaction in an amount of from 2 wt% to 10 wt% based on the dry weight of the purification residue.

14. The method according to Claim 1, comprising:
(a) continuously feeding a purification residue stream to a reaction vessel, containing one or more of the trimethylolpropane formals;
(b) providing water, an acid and an oxidizing agent to the reaction vessel such that a reaction mixture including the purification residue, water, acid and oxidizing agent is formed;
(c) maintaining the reaction mixture in the reaction vessel at a time and temperature such that one or more of said TMP-formals are hydrolyzed to thereby enrich the reaction mixture with reclaimed trimethylolpropane; and
(d) continuously withdrawing a reclaimed trimethylolpropane stream from the reaction vessel.

15. The process according to Claim 14, wherein the reclaimed trimethylolpropane stream from step (d) is subjected to a distillation to obtain trimethylolpropane containing light ends and a distillation residue.

16. The method according to Claim 15, wherein the reclaimed trimethylolpropane stream is combined with another trimethylolpropane stream to provide a combined trimethylolpropane stream prior to distillation.

17. The method according to Claim 15, wherein formate is extracted from the combined trimethylolpropane stream prior to distillation.

18. The method according to Claim 1 comprising:
(a) distilling a trimethylolpropane containing stream to provide an overhead product stream and a purification residue stream;
(b) feeding the purification residue stream to a reaction vessel, said purification residue stream containing one or more of the trimethylolpropane formals;
(c) continuously providing water, an acid and an oxidizing agent to the reaction vessel such that a reaction mixture including the purification residue, water, acid and oxidizing agent is formed;
(d) maintaining the reaction mixture in the reaction vessel at a time and temperature such that one or more of said trimethylol formals are hydrolyzed to thereby enrich the reaction mixture with reclaimed trimethylolpropane;
(e) withdrawing a reclaimed trimethylolpropane stream from the reaction vessel; and
(f) recycling reclaimed trimethylolpropane to step (a).

19. The method according to Claim 1, implemented with a purification system for recovering trimethylolpropane from a raw product stream comprising trimethylolpropane and formate, the purification system comprising:
(a) an extraction system adapted to receive the raw product stream and provide aqueous output containing formate and an organic output containing trimethylolpropane;
(b) a distillation tower adapted to receive organic output of the extraction system and provide trimethylolpropane and light ends as overhead and purification residue;
**characterized in that** the system includes:
(c) means for providing water, an oxidizing agent, and acid to the purification residue to provide an aqueous residue mixture;
(d) a reactor adapted for receiving the aqueous residue mixture and maintaining the reaction mixture in the reaction vessel at a time and temperature such that one or more of said TMP-formals are hydrolyzed to thereby enrich the reaction mixture with reclaimed trimethylolpropane; and
(e) a recycle system for recycling reclaimed trimethylolpropane from the reactor vessel to the extraction system of part (a) or for recycling reclaimed trimethylolpropane from the reactor to the distillation tower of part (b).

## Patentansprüche

1. Verfahren zur Wiedergewinnung von Trimethylolpropan aus Reinigungsrückstand, umfassend das Hydrolysieren eines Reinigungsrückstands mit einem wässrigen Medium in Gegenwart von (i) einer Säure und (ii) einem Oxidationsmittel zur Wiedergewinnung von Trimethylolpropan aus einem oder mehreren TMP-Formalen, ausgewählt aus:
monozyklischem TMP-Formal (MCF); Formel I: 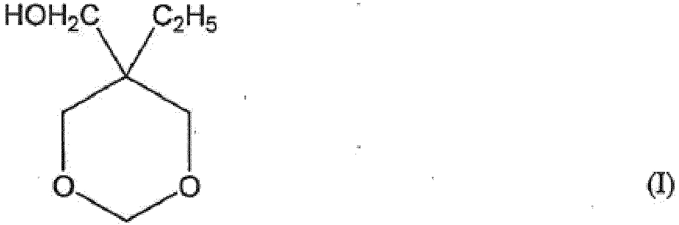
monolinearem Bis-TMP-Formal (MBLF); Formel II:
[C₂H₅C(CH₂OH)₂CH₂O]₂CH₂ (II)
Methyl-(monolinearem) TMP-Formal; Formel III:
C₂H₅C(CH₂OH)₂CH₂OCH₂OCH₃ (III)
Methyl-(bislinearem) TMP-Formal; Formel IV:
C₂H₅C(CH₂OH)₂CH₂OCH₂OCH₂OCH₃ (III)
und dem zyklischen Formal von Di-TMP; Formel V: 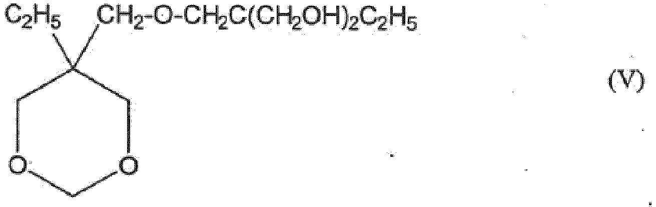 wobei das Oxidationsmittel der Hydrolysereaktion in einer Menge von 2 Gew.-% bis 40 Gew.-%, bezogen auf das Trockengewicht des Reinigungsrückstands, zugeführt wird.

2. Verfahren nach Anspruch 1, umfassend das Hydrolysieren von monolinearem Bis-TMP-Formal (MBLF); Formel II:
[C₂H₅C(CH₂OH)₂CH₂O]₂CH₂ (II)
in Gegenwart von (i) einer Säure und (ii) einem Oxidationsmittel.

3. Verfahren nach Anspruch 1, bei dem die Säure aus Schwefelsäure, Phosphorsäure, Salzsäure, Sulfonsäure, organischen Säuren oder säurefunktionellem Ionenaustauscherharz ausgewählt wird.

4. Verfahren nach Anspruch 1, bei dem es sich bei der Säure um Ameisensäure handelt.

5. Verfahren nach Anspruch 1, bei dem es sich bei der Säure um ein säurefunktionelles Ionenaustauscherharz handelt.

6. Verfahren nach Anspruch 1, bei dem die Hydrolysereaktion bei einem pH-Wert von 1 bis 4 durchgeführt wird.

7. Verfahren nach Anspruch 1, ferner umfassend das Neutralisieren des wässrigen Mediums mit einem Neutralisationsmittel.

8. Verfahren nach Anspruch 1, bei dem die Hydrolysereaktion über eine Reaktionsdauer von 1,5 bis 4,5 Stunden durchgeführt wird.

9. Verfahren nach Anspruch 1, bei dem die Säure dem wässrigen Reaktionsmedium in einer Menge von 0,5 Gew.-% bis 20 Gew.-%, bezogen auf das Gesamtgewicht des wässrigen Mediums, zugeführt wird.

10. Verfahren nach Anspruch 1, bei dem Wasser dem Reaktionsmedium in einem Gewichtsverhältnis von Wasser zu organischem Gemisch von 1 W/W bis 3 W/W zugeführt wird.

11. Verfahren nach Anspruch 1, bei dem die Hydrolysetemperatur im Bereich von 70°C bis 95°C gehalten wird.

12. Verfahren nach Anspruch 1, bei dem die Hydrolysereaktion über eine Reaktionsdauer von 1 Stunde bis 7,5 Stunden durchgeführt wird.

13. Verfahren nach Anspruch 1, bei dem das Oxidationsmittel der Hydrolysereaktion in einer Menge von 2 Gew.-% bis 10 Gew.-%, bezogen auf das Trockengewicht des Reinigungsrückstands, zugeführt wird.

14. Verfahren nach Anspruch 1, umfassend:
(a) kontinuierliches Einspeisen eines Reinigungsrückstandsstroms, der ein oder mehrere der Trimethylpropanformale enthält, in ein Reaktionsgefäß;
(b) Versorgen des Reaktionsgefäßes mit Wasser, einer Säure und einem Oxidationsmittel derart, dass ein den Reinigungsrückstand, Wasser, Säure und Oxidationsmittel enthaltendes Reaktionsgemisch gebildet wird;
(c) Halten des Reaktionsgemischs im Reaktionsgefäß über einen solchen Zeitraum und bei einer solchen Temperatur, dass ein oder mehrere der TMP-Formale hydrolysiert werden, wodurch das Reaktionsgemisch mit wiedergewonnenem Trimethylolpropan angereichert wird; und
(d) kontinuierliches Abziehen eines Stroms von wiedergewonnenem Trimethylolpropan aus dem Reaktionsgefäß.

15. Verfahren nach Anspruch 14, bei dem der Strom von wiedergewonnenem Trimethylolpropan aus Schritt (d) einer Destillation unterworfen wird, bei der man Trimethylolpropan enthaltende Leichtsieder und einen Destillationsrückstand erhält.

16. Verfahren nach Anspruch 15, bei dem der Strom von wiedergewonnenem Trimethylolpropan vor der Destillation mit einem anderen Trimethylolpropanstrom zu einem kombinierten Trimethylolpropanstrom kombiniert wird.

17. Verfahren nach Anspruch 15, bei dem vor der Destillation aus dem kombinierten Trimethylolpropanstrom Formiat extrahiert wird.

18. Verfahren nach Anspruch 1, umfassend:
(a) Destillieren eines Trimethylolpropan enthaltenden Stroms zur Bereitstellung eines Kopfproduktstroms und eines Reinigungsrückstandsstroms;
(b) Einspeisen des Reinigungsrückstandsstroms in ein Reaktionsgefäß, wobei der Reinigungsrückstandsstrom ein oder mehrere der Trimethylpropanformale enthält;
(c) kontinuierliches Versorgen des Reaktionsgefäßes mit Wasser, einer Säure und einem Oxidationsmittel derart, dass ein den Reinigungsrückstand, Wasser, Säure und Oxidationsmittel enthaltendes Reaktionsgemisch gebildet wird;
(d) Halten das Reaktionsgemischs im Reaktionsgefäß über einen solchen Zeitraum und bei einer solchen Temperatur, dass ein oder mehrere der Trimethylolformale hydrolysiert werden, wodurch das Reaktionsgemisch mit wiedergewonnenem Trimethylolpropan angereichert wird; und
(e) Abziehen eines Stroms von wiedergewonnenem Trimethylolpropan aus dem Reaktionsgefäß; und
(f) Rezyklieren von wiedergewonnenem Trimethylolpropan zum Schritt (a).

19. Verfahren nach Anspruch 1, ausgeführt mit einem Reinigungssystem zur Gewinnung von Trimethylolpropan aus einem Rohproduktstrom, der Trimethylolpropan und Formiat umfasst, wobei das Reinigungssystem Folgendes umfasst:
(a) ein Extraktionssystem, das zum Empfangen des Rohproduktstroms und zum Bereitstellen eines wässrigen Austrags, der Formiat enthält, und eines organischen Austrags, der Trimethylolpropan enthält, ausgelegt ist;
(b) einen Destillationsturm, der zum Empfangen von organischem Austrag des Extraktionssystems und zum Bereitstellen von Trimethylolpropan und Leichtsiedern als Kopfprodukt und Reinigungsrückstand ausgelegt ist;
**dadurch gekennzeichnet, dass** das System Folgendes einschließt:
(c) Möglichkeiten zum Versorgen des Reinigungsrückstands mit Wasser, einem Oxidationsmittel und Säure zur Bereitstellung eines wässrigen Rückstandsgemischs;
(d) einen Reaktor, der zur Aufnahme des wässrigen Rückstandsgemischs und zum Halten des Reaktionsgemischs im Reaktionsgefäß über einen solchen Zeitraum und bei einer solchen Temperatur, dass ein oder mehrere der TMP-Formale hydrolysiert werden, ausgelegt ist, wodurch das Reaktionsgemisch mit wiedergewonnenem Trimethylolpropan angereichert wird; und
(e) ein Rezyklierungssystem zum Rezyklieren von wiedergewonnenem Trimethylolpropan aus dem Reaktionsgefäß in das Extraktionssystem von Teil (a) oder zum Rezyklieren von wiedergewonnenem Trimethylolpropan aus dem Reaktor in den Destillationsturm von Teil (b).

## Revendications

1. Procédé pour régénérer du triméthylolpropane à partir d'un résidu de purification, comprenant l'hydrolyse d'un résidu de purification avec un milieu aqueux en présence (i) d'un acide et (ii) d'un agent oxydant, pour régénérer le triméthylolpropane à partir d'un ou plusieurs TMP-formals choisis parmi :
le TMP-formal monocyclique (MCF) ; formule I : 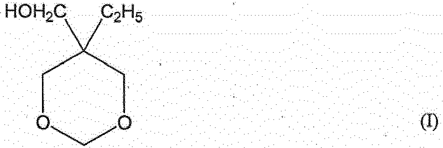
le bis-TMP-formal monolinéaire (MBLF) ; formule II :
[C₂H₅C(CH₂OH)₂CH₂O]₂CH₂ (II)
le méthyl-TMP-formal (monolinéaire) ; formule III :
C₂H₅C(CH₂OH)₂CH₂OCH₂OCH₃ (III)
le méthyl-TMP-formal (bislinéaire) ; formule IV :
C₂H₅C(CH₂OH)₂CH₂OCH₂OCH₂OCH₃ (IV)
et le formal cyclique du di-TMP ; formule V : 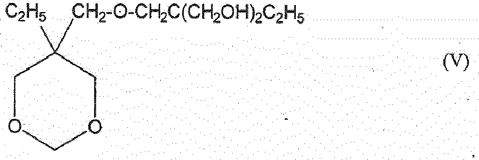 l'agent oxydant étant amené à la réaction d'hydrolyse en une quantité de 2 % en poids à 40 % en poids par rapport au poids sec du résidu de purification.

2. Procédé selon la revendication 1, comprenant l'hydrolyse du bis-TMP-formal monolinéaire (MBLF) ; formule II :
[C₂H₅C(CH₂OH)₂CH₂O]₂CH₂ (II)
en présence (i) d'un acide, et (ii) d'un agent oxydant.

3. Procédé selon la revendication 1, dans lequel ledit acide est choisi parmi l'acide sulfurique, l'acide phosphorique, l'acide chlorhydrique, un acide sulfonique, des acides organiques ou des résines échangeuses d'ions à fonctionnalité acide.

4. Procédé selon la revendication 1, dans lequel ledit acide est l'acide formique.

5. Procédé selon la revendication 1, dans lequel ledit acide est une résine échangeuse d'ions à fonctionnalité acide.

6. Procédé selon la revendication 1, dans lequel la réaction d'hydrolyse est mise en oeuvre à un pH de 1 à 4.

7. Procédé selon la revendication 1, comprenant en outre la neutralisation dudit milieu aqueux avec un agent de neutralisation.

8. Procédé selon la revendication 1, dans lequel la réaction d'hydrolyse est mise en oeuvre pendant un temps de réaction de 1,5 à 4,5 heures.

9. Procédé selon la revendication 1, dans lequel l'acide est amené au milieu réactionnel aqueux en une quantité de 0,5 % en poids à 20 % en poids par rapport au poids total du milieu aqueux.

10. Procédé selon la revendication 1, dans lequel l'eau est amenée au milieu réactionnel selon un rapport en poids eau:mélange organique de 1 p/p à 3 p/p.

11. Procédé selon la revendication 1, dans lequel la température de l'hydrolyse est maintenue dans la plage de 70°C à 95°C.

12. Procédé selon la revendication 1, dans lequel la réaction d'hydrolyse est mise en oeuvre pendant un temps de réaction de 1 heure à 7,5 heures.

13. Procédé selon la revendication 1, dans lequel l'agent oxydant est amené à la réaction d'hydrolyse en une quantité de 2 % en poids à 10 % en poids par rapport au poids sec du résidu de purification.

14. Procédé selon la revendication 1, comprenant :
(a) l'introduction en continu d'un courant de résidu de purification dans un récipient réactionnel contenant un ou plusieurs des triméthylolpropane-formals ;
(b) l'amenée d'eau, d'un acide et d'un agent oxydant au récipient réactionnel, de telle sorte qu'il se forme un mélange réactionnel comprenant le résidu de purification, l'eau, l'acide et l'agent oxydant ;
(c) le maintien du mélange réactionnel dans le récipient réactionnel pendant un temps et à une température tels qu'un ou plusieurs desdits TMP-formals soient hydrolysés, de façon à enrichir de ce fait le mélange réactionnel en le triméthylolpropane régénéré ; et
(d) le soutirage en continu, à partir du récipient réactionnel, du courant de triméthylolpropane régénéré.

15. Procédé selon la revendication 14, dans lequel le courant de triméthylolpropane régénéré de l'étape (d) est soumis à une distillation pour obtenir un triméthylolpropane contenant des fractions légères et un résidu de distillation.

16. Procédé selon la revendication 15, dans lequel le courant de triméthylolpropane régénéré est combiné avant une distillation à un autre courant de triméthylolpropane, pour donner un courant de triméthylolpropane combiné.

17. Procédé selon la revendication 15, dans lequel un formiate est extrait, avant la distillation, du courant de triméthylolpropane combiné.

18. Procédé selon la revendication 1, comprenant :
(a) la distillation d'un courant contenant du triméthylolpropane pour donner un courant de produit de tête et un courant de résidu de purification ;
(b) l'introduction du courant de résidu de purification dans un récipient réactionnel, ledit courant de résidu de purification contenant un ou plusieurs des triméthylolpropane-formals ;
(c) l'amenée continue d'eau, d'un acide et d'un agent oxydant au récipient réactionnel de telle sorte qu'il se forme un mélange réactionnel comprenant le résidu de purification, l'eau, l'acide et l'agent oxydant ;
(d) le maintien du mélange réactionnel dans le récipient réactionnel pendant un temps ou à une température tels qu'un ou plusieurs desdits triméthylol-formals soient hydrolysés de façon à enrichir de ce fait le mélange réactionnel en triméthylolpropane régénéré ;
(e) le soutirage, à partir du récipient réactionnel, d'un courant de triméthylolpropane régénéré ; et
(f) le recyclage vers l'étape (a) du triméthylolpropane régénéré.

19. Procédé selon la revendication 1, mis en oeuvre dans un système de purification pour récupérer le triméthylolpropane à partir d'un courant de produit brut comprenant du triméthylolpropane et un formiate, le système de purification comprenant :
(a) un système d'extraction conçu pour recevoir le courant de produit brut et fournir un produit de sortie aqueux contenant le formiate et un produit de sortie organique contenant le triméthylolpropane ;
(b) une tour de distillation, conçue pour recevoir le produit de sortie organique du système d'extraction et fournir du triméthylolpropane et des fractions légères en tant que produit de tête et résidu de purification ;
**caractérisé en ce que** le système comprend :
(c) un moyen pour amener de l'eau, un agent oxydant et un acide au résidu de purification, pour fournir un mélange de résidus aqueux ;
(d) un réacteur conçu pour recevoir le mélange de résidus aqueux et maintenir le mélange réactionnel dans le récipient réactionnel pendant un temps et à une température tels qu'un ou plusieurs desdits TMP-formals soit hydrolysé, pour enrichir de ce fait le mélange réactionnel en triméthylolpropane régénéré ; et
(e) un système de recyclage pour recycler le triméthylolpropane régénéré, du récipient réactionnel au système d'extraction de la partie (a), ou pour recycler le triméthylolpropane régénéré, du réacteur vers la tour de distillation de la partie (b).
